# EUROPÄISCHE PATENTSCHRIFT

(11) **EP 0 603 649 B1**
(45) Veröffentlichungstag und Bekanntmachung des Hinweises auf die Patenterteilung: **10.07.1996**
(21) Anmeldenummer: 93119766.9
(22) Anmeldetag: 08.12.1993
(51) Int. Cl.: C07D 405/06, A61K 31/44, C07D 213/64

(54) **Substituierte 4-Phenyl-pyridone und 4-Phenyl-2-alkoxypyridine als HMG-CoA-Reductase-Inhitoren**
Substituted 4-phenyl-pyridones and 4-phenyl-2-alkoxypyridines as HMG-CoA reductase inhibitors
4-Phenylpyridones et 4-phenyl-2-alkoxypyridines substituées comme inhibiteurs de réductase de HMG-CoA

(30) Priorität: 21.12.1992 DE 4243278; 28.06.1993 DE 4321421
(43) Veröffentlichungstag der Anmeldung: 29.06.1994
(73) Patentinhaber: BAYER AG, 51368 Leverkusen (DE)
(72) Erfinder: Angerbauer, Rolf, Dr., D-42113 Wuppertal (DE); Fey, Peter, Dr., D-42111 Wuppertal (DE); Hübsch, Walter, Dr., D-42113 Wuppertal (DE); Philipps, Thomas, Dr., D-51065 Köln (DE); Bischoff, Hilmar, Dr., D-42113 Wuppertal (DE); Krause, Hans Peter, Dr., D-58332 Schwelm (DE); Petersen von Gehr, Jörg, Dr., D-44795 Bochum (DE); Schmidt, Delf, Dr., D-42113 Wuppertal (DE)

(56) Entgegenhaltungen:
- EP-A- 0 306 929
- EP-A- 0 373 423
- JOURNAL OF MEDICINAL CHEMISTRY Bd. 33, Nr. 1 , 1990 , WASHINGTON US Seiten 52 - 60 G. BECK ET AL.

## Beschreibung

Die Erfindung betrifft substituierte 4-Phenyl-pyridone und 4-Phenyl-2-alkoxypyridine, Verfahren zu ihrer Herstellung, sowie ihre Verwendung in Arzneimitteln.

Es ist bekannt, daß aus Pilzkulturen isolierte Lacton-Derivate Inhibitoren der 3-Hydroxy-3-methyl-glutaryl Coenzym A Reduktase (HMG-CoA-Reduktase) sind [Mevinolin, EP 22 478; US 4 231 938].

Außerdem ist bekannt, daß Pyridin-substituierte Dihydroxyheptensäuren Inhibitoren der HMG-CoA-Reduktase sind [EP 325 130; EP 307 342; EP 306 929].

Aus EP-A-O 373 423 sind bereits substituierte Pyridone mit HMG-CoA-Reduktase inhibierender Wirkung bekannt, die als funktionelle Seitenkette eine Dihydroxycarbonsäure-Seitenkette besitzen.

Die vorliegende Erfindung betrifft jetzt substituierte 4-Phenyl-pyridone und 4-Phenyl-2-alkoxypyridine der allgemeinen Formel (I) 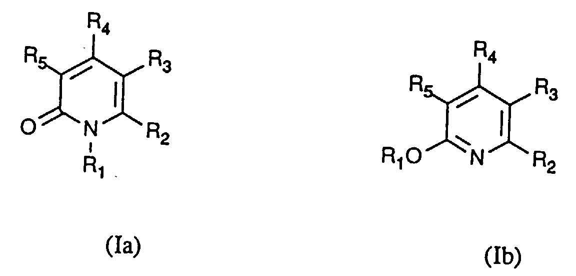 in welcher
- R¹: für geradkettiges oder verzweigtes Alkyl mit bis zu 8 Kohlenstoffatomen steht,
- R²: für Cycloalkyl mit 3 bis 7 Kohlenstoffatomen steht, oder für geradkettiges oder verzweigtes Alkyl mit bis zu 8 Kohlenstoffatomen steht,
- R³: für einen Rest der Formel 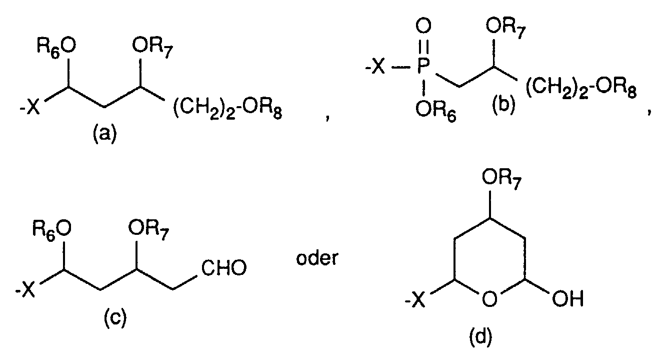 steht,
worin
- X: die Gruppe -CH₂-CH₂-, -CH=CH- oder -C≡C- bedeutet,
und
- R⁶, R⁷ und R⁸: gleich oder verschieden sind und Wasserstoff oder einen Rest der Formel -CO-R⁹ oder -COOR¹⁰ bedeuten,
worin
- R⁹ und R¹⁰: gleich oder verschieden sind und geradkettiges oder verzweigtes Alkyl mit bis zu 8 Kohlenstoffatomen oder Phenyl bedeuten,
oder
- R⁶ und R⁷: gemeinsam einen Rest der Formel 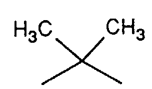 bilden,
- R⁴: für Phenyl steht, das gegebenenfalls bis zu 2-fach gleich oder verschieden durch Halogen, Trifluormethyl, Methoxy, Phenoxy oder geradkettiges oder verzweigtes Alkyl mit bis zu 8 Kohlenstoffatomen substituiert ist,
- R⁵: die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 8 Kohlenstoffatomen steht, oder
für geradkettiges oder verzweigtes Alkyl mit bis zu 8 Kohlenstoffatomen steht, das gegebenenfalls durch Halogen, Hydroxy, durch geradkettiges oder verzweigtes Alkoxy mit bis zu 8 Kohlenstoffatomen, oder durch eine Gruppe der Formel -O-(CH₂)ₐ-R¹¹ oder -O-CO-R¹² substituiert ist,
worin
- a: eine Zahl 0 oder 1 bedeutet,
- R¹¹: Phenyl oder Benzyl bedeutet, die gegebenenfalls in Aromaten bis zu 2-fach gleich oder verschieden durch Halogen, Trifluormethyl, Cyano, Nitro oder durch geradkettiges oder verzweigtes Alkyl mit bis zu 6 Kohlenstoffatomen substituiert sind,
- R¹²: die oben angegebene Bedeutung von R¹¹ hat und mit dieser gleich oder verschieden ist, oder
geradkettiges oder verzweigtes Alkyl mit bis zu 8 Kohlenstoffatomen bedeutet,
oder
- R⁵: für einen Rest der Formel -CH=N-O-R¹³ steht,
worin
R¹³ die oben angegebene Bedeutung von R¹² hat und mit dieser gleich oder verschieden ist.

Die erfindungsgemäßen Verbindungen haben jeweils in Abhängigkeit der unter R³ und/oder R⁵ aufgeführten Seitenketten, 1 oder 2 asymmetrische Kohlerstoffatomen, an denen die Reste -OR⁶ und -OR⁷ gebunden sind. Sie können daher in verschiedenen stereochemischen Formen existieren.

Die Erfindung betrifft sowohl die einzelnen Isomeren als auch deren Mischungen. So können die erfindungsgemäßen Stoffe je nach der relativen Stellung der Reste -OR⁶ / -OR⁷ in der Erythro-Konfiguration oder in der Threo-Konfiguration vorliegen.

Dies soll für den Substituenten R³/R⁵ mit der Bedeutung a) wie folgt beispielhaft erläutert werden: 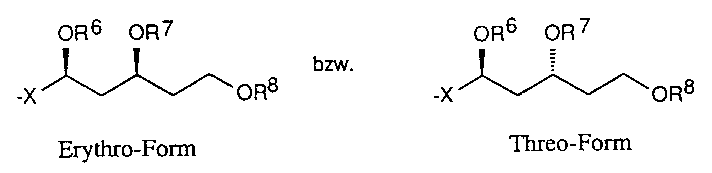 Sowohl von den Stoffen in Threo- als auch in Erythro-Konfiguration existieren wiederum jeweils zwei Enantiomere.

Bevorzugt sind jeweils die Erythro-Formen.

Darüber hinaus können die erfindungsgemäßen Stoffe aufgrund der Doppelbindung (X = -CH=CH-) in den E-Konfiguration oder der Z-Konfiguration vorliegen. Bevorzugt sind diejenigen Verbindungen, die E-konfiguriert sind.

Außerdem stehen die Aldehydreste (Subst. (c)) jeweils im Gleichgewicht mit den entsprechenden Hydroxypyranen (Subst. (d)).

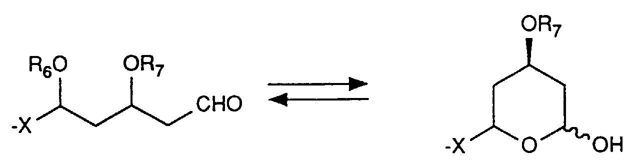

Bevorzugt sind Verbindungen der allgemeinen Formel (I)
in welcher
- R¹: für geradkettiges oder verzweigtes Alkyl mit bis zu 6 Kohlenstoffatomen steht,
- R²: für Cyclopropyl, Cyclopentyl oder Cyclohexyl steht, oder für geradkettiges oder verzweigtes Alkyl mit bis zu 6 Kohlenstoffatomen steht,
- R³: für einen Rest der Formel 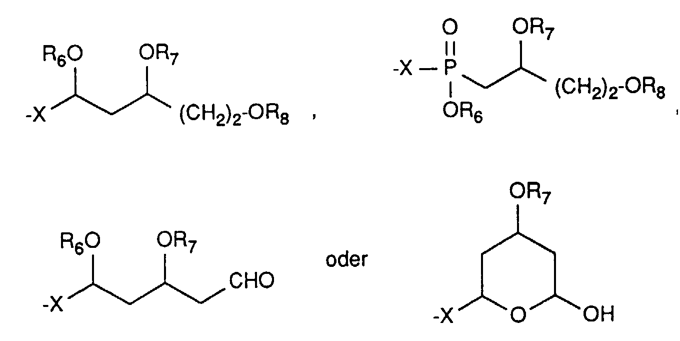 steht,
worin
- X: die Gruppe -CH₂-CH₂-, -CH=CH- oder -C≡C- bedeutet,
- R⁶, R⁷ und R⁸: gleich oder verschieden sind und Wasserstoff oder einen Rest der Formel -CO-R⁹ oder -COO-R¹⁰ bedeuten,
worin
- R⁹ und R¹⁰: gleich oder verschieden sind und geradkettiges oder verzweigtes Alkyl mit bis zu 6 Kohlenstoffatomen oder Phenyl bedeuten,
oder
- R⁶ und R⁷: gemeinsam einen Rest der Formel 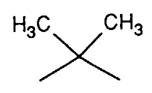 bilden,
- R⁴: für Phenyl steht, das gegebenenfalls bis zu 2-fach gleich oder verschieden durch Fluor, Chlor, Brom, Trifluormethyl oder durch geradkettiges oder verzweigtes Alkyl mit bis zu 6 Kohlenstoffatomen substituiert ist,
- R⁵: die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 6 Kohlenstoffatomen steht, oder
für geradkettiges oder verzweigtes Alkyl mit bis zu 6 Kohlenstoffatomen steht, das gegebenenfalls durch Fluor, Chlor, Brom, Hydroxy oder durch geradkettiges oder verzweigtes Alkoxy mit bis zu 6 Kohlenstoffatomen substituiert ist, oder das durch eine Gruppe der Formel -O-(CH₂)ₐ-R¹¹ oder -O-CO-R¹² substituiert ist,
worin
- a: eine Zahl 0 oder 1 bedeutet,
und
- R¹¹: Phenyl oder Benzyl bedeutet, die in Aromaten gegebenenfalls durch Fluor, Chlor, Brom, Trifluormethyl, Cyano, Nitro oder durch geradkettiges oder verzweigtes Alkyl mit bis zu 4 Kohlenstoffatomen substituiert sind,
- R¹²: die oben angegebene Bedeutung von R¹¹ hat und mit dieser gleich oder verschieden ist, oder
geradkettiges oder verzweigtes Alkyl mit bis zu 6 Kohlenstoffatomen bedeutet,
oder
- R⁵: für einen Rest der Formel -CH=N-O-R¹³ steht,
worin
R¹³ die oben angegebene Bedeutung von R¹² hat und mit dieser gleich oder verschieden ist.

Besonders bevorzugt sind Verbindungen der allgemeinen Formel (I),
in welcher
- R¹: für geradkettiges oder verzweigtes Alkyl mit bis zu 4 Kohlenstoffatomen steht,
- R²: für Cyclopropyl oder für geradkettiges oder verzweigtes Alkyl mit bis zu 4 Kohlenstoffatomen steht,
- R³: für einen Rest der Formel 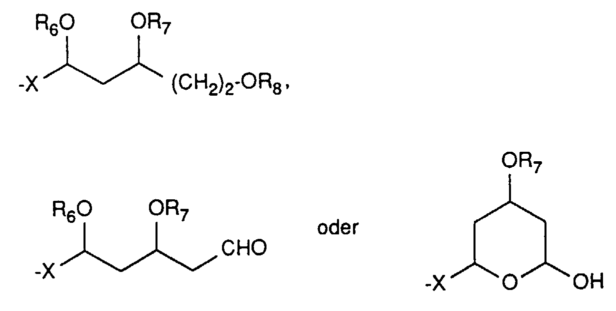 steht,
worin
- X: die Gruppe -CH₂-CH₂- oder -CH=CH- bedeutet,
- R⁶, R⁷ und R⁸: gleich oder verschieden sind und Wasserstoff oder einen Rest der Formel -CO-R⁹ bedeuten,
worin
R⁹ geradkettiges oder verzweigtes Alkyl mit bis zu 4 Kohlenstoffatomen bedeutet,
- R⁴: für Phenyl steht, das gegebenenfalls durch Fluor, Trifluormethyl oder durch geradkettiges oder verzweigtes Alkyl mit bis zu 4 Kohlenstoffatomen substituiert ist,
- R⁵: die oben angegebene Bedeutung von R³ hat und mit dieser gleich oder verschieden ist, oder
für Wasserstoff oder
für geradkettiges oder verzweigtes Alkyl mit bis zu 4 Kohlenstoffatomen steht, das gegebenenfalls durch Hydroxy oder durch geradkettiges oder verzweigtes Alkoxy mit bis zu 4 Kohlenstoffatomen substituiert ist.

Ganz besonders bevorzugt sind Verbindungen der allgemeinen Formel (I),
in welcher
- R¹: für geradkettiges oder verzweigtes Alkyl mit bis zu 4 Kohlenstoffatomen steht,
- R²: für Cyclopropyl oder für geradkettiges oder verzweigtes Alkyl mit bis zu 4 Kohlenstoffatomen steht,
- R³: für einen Rest der Formel 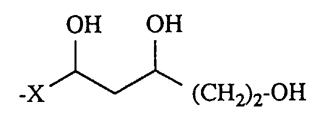 steht,
wobei
- X: die Gruppe -CH₂-CH₂- oder -CH=CH- bedeutet,
- R⁴: für Phenyl steht, das gegebenenfalls durch Fluor substituiert ist, und
- R⁵: die oben angegebene Bedeutung von R³ hat oder
für gradkettiges oder verzweigtes Alkyl mit bis zu 4 Kohlenstoffatomen steht, das gegebenenfalls durch Hydroxy oder Alkoxy mit bis zu vier Kohlenstoffatomen substituiert ist.

Insbesondere bevorzugt sind die erfindungsgemäßen Verbindungen der allgemeinen Formel (I),
in welcher
- R¹: für Methyl steht
und
- R³: für den Rest der Formel 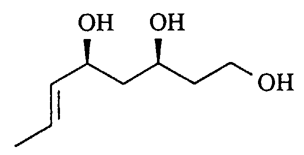 steht.

Außerdem wurde ein Verfahren zur Herstellung der erfindungsgemäßen Verbindungen der allgemeinen Formel (I) gefunden, das dadurch gekennzeichnet ist, daß man

Pyridone oder 2-Alkoxypyridine der allgemeinen Formel (II) 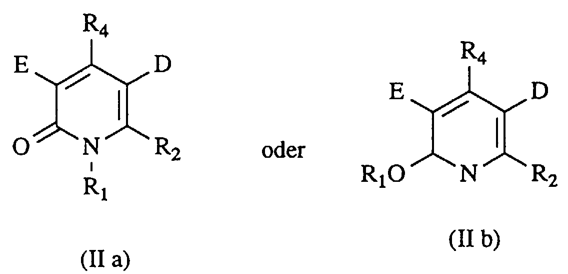 in welcher
- R¹, R² und R⁴: die angegebene Bedeutung haben
und
- D: für einen Rest der Formel 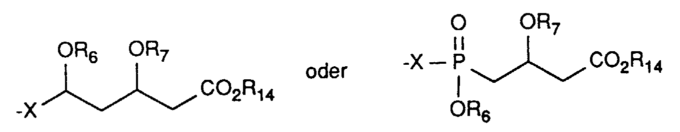 steht,
worin
- A, B, R⁶ und R⁷: die angegebene Bedeutung haben
- R¹⁴: für C₁-C₆-Alkyl steht
und
- E: entweder ebenfalls die oben angegebene Bedeutung von D hat oder die oben angegebene Bedeutung von R⁵ hat,
in inerten Lösemitteln, unter Schutzgasatmosphäre, gegebenenfalls über die Stufe des Aldehyds, mit Reduktionsmitteln reduziert,
und im Fall, daß -X- für die -CH₂-CH₂-Gruppe steht, die Ethengtuppe (X = -CH=CH-) oder die Ethingruppe (X = -C≡C-) schrittweise nach üblichen Methoden hydriert, und gegebenenfalls die Isomeren trennt.

Das erfindungsgemäße Verfahren kann durch folgendes Formelschema beispielhaft erläutert werden:

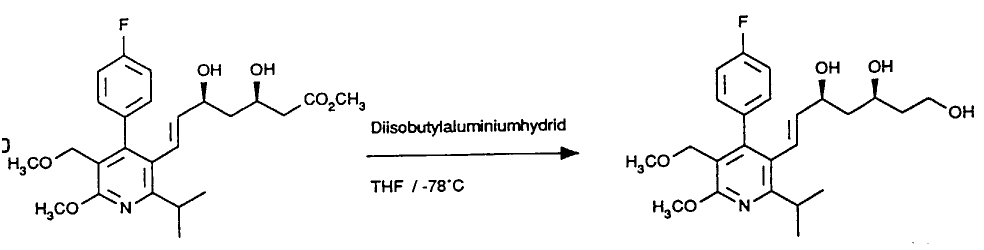

Als Lösemittel für die Reduktion eignen sich im allgemeinen die üblichen organischen Lösemittel. Bevorzugt sind Ether wie Diethylether, Tetrahydrofuran oder Dioxan. Besonders bevorzugt ist Tetrahydrofuran.

Als Reduktionsmittel eignen sich komplexe Metallhydride, wie beispielsweise Lithiumaluminiumhydrid, Natriumcyanoborhydrid, Natriumaluminiumhydrid, Diisobutylaluminiumhydrid oder Natrium-bis-(2-methoxyethoxy)dihydroaluminat. Bevorzugt ist Diisobutylaluminiumhydrid.

Das Reduktionsmittel wird im allgemeinen in einer Menge von 4 mol bis 10 mol, bevorzugt von 4 mol bis 5 mol bezogen auf 1 mol der Verbindungen der allgemeinen Formel(II) eingesetzt.

Die Reduktion verläuft im allgemeinen in einem Temperaturbereich von -78°C bis +50°C, bevorzugt von -78°C bis 0°C, besonders bevorzugt bei -78°C, jeweils in Abhängigkeit von der Wahl des Reduktionsmittels und Lösemittels.

Die Reduktion verläuft im allgemeinen bei Normaldruck, es ist aber auch möglich bei erhöhtem oder erniedrigtem Druck zu arbeiten.

Die Cyclisierung der Aldehyde zu den entsprechenden Hydroxy-Pyranen erfolgt im allgemeinen bei Raumtemperatur oder durch Erhitzen in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt wird Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40°C bis +100°C, bevorzugt von -25°C bis +50°C durchgeführt.

Die Hydrierung der Doppelbindung erfolgt nach üblichen Methoden mit Wasserstoff in Anwesenheit von Edelmetallkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Gegebenenfalls erfolgt die Reduktion der Dreifach- bzw. Doppelbindung auch bei der oben aufgeführten Reduktion der Estergruppe.

Die Verbindungen der allgemeinen Formel (II) in denen D und/oder E für den Rest der Formel 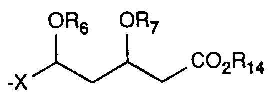 steht, werden hergestellt
indem man
Ketone der allgemeinen Formeln IIIa, b 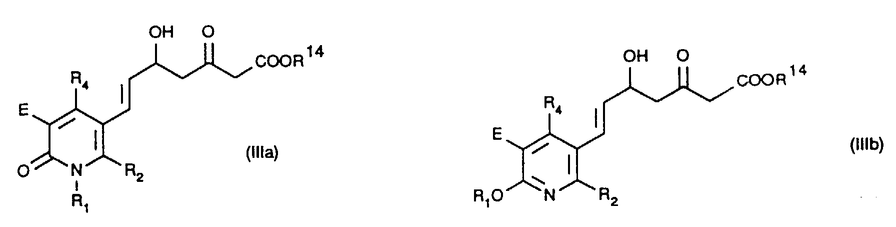 in welcher
R¹ bis R⁴ und R¹⁴die angegebene Bedeutung haben
reduziert und gegebenenfalls Isomere trennt.

Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-boranaten, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispielsweise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -80°C bis +30°C, bevorzugt von -78°C bis 0°C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol, bezogen auf 1 mol der Ketoverbindung, eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Die als Ausgangsstoffe eingesetzten Ketone IIIa, b werden hergestellt, indem man Aldehyde der Formeln IVa, b 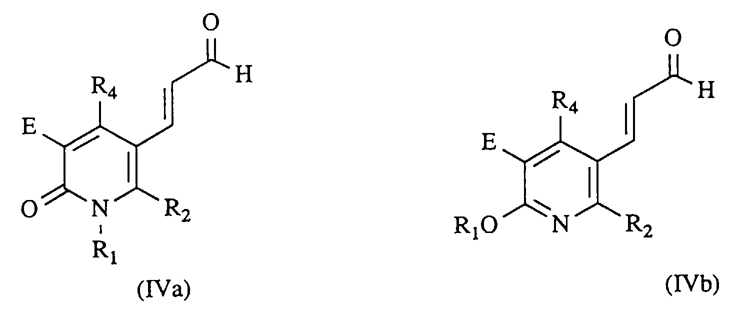 in welcher
R¹ bis R⁴ die angegebene Bedeutung haben,
in inerten Lösemitteln mit Acetessigestern der allgemeinen Formel V 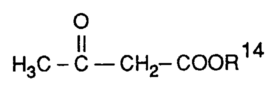 in welcher
R¹⁴ die angegebene Bedeutung hat,
in Anwesenheit von Basen umsetzt.

Als Basen kommen hierbei die üblichen stark basischen Verbindungen in Frage. Hierzu gehören bevorzugt lithiumorganische Verbindungen wie beispielsweise n-Butyllithium, sec. Butyllithium, tert.Butyllithium oder Phenyllithium oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt wird n-Butyllithium oder Natriumhydrid oder deren Gemisch eingesetzt.

Eventuell sind Zusätze von Metallhalogeniden wie z.B. Magnesiumchlorid, Zinkchlorid oder Zinkbromid vorteilhaft. Besonders bevorzugt ist der Zusatz von Zinkhalogeniden.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erdölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80°C bis +50°C, bevorzugt von -20°C bis Raumtemperatur durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (V) sind bekannt oder können nach bekannten Methoden hergestellt werden.

Als Acetessigester für das erfindungsgemäße Verfahren seien beispielsweise genannt:
Acetessigsäuremethylester, Acetessigsäureethylester, Acetessigsäurepropylester, Acetessigsäureisopropylester.

Die Herstellung der als Ausgangsstoffe eingesetzten Aldehyde der allgemeinen Formel IVa, b soll im folgenden beispielhaft für die Verbindungen des Typs (IVb) erläutert werden.

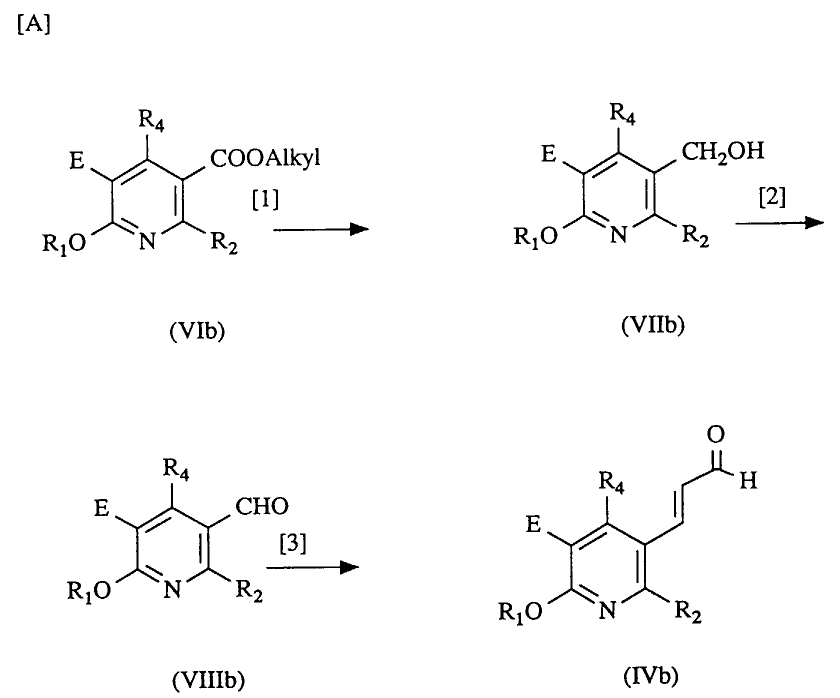

Hierbei werden gemäß Schema A Verbindungen der Formel VIb im ersten Schritt [1] in inerten Lösemitteln wie Ethern, beispielsweise Diethylether, Tetrahydrofuran oder Dioxan, vorzugsweise Tetrahydrofuran, mit Metallhydriden als Reduktionsmittel, beispielsweise Lithiumaluminiumhydrid, Natriumcyanoborhydrid, Natriumaluminiumhydrid, Diisobutylaluminiumhydrid oder Natrium-bis-(2-methoxyethoxy)-dihydroaluminat, in Temperaturbereichen von -70°C bis +100°C, vorzugsweise von -70°C bis Raumtemperatur, bzw. von Raumtemperatur bis +70°C je nach verwendetem Reduktionsmittel zu den Hydroxymethylverbindungen reduziert. Vorzugsweise wird die Reduktion mit Diisobutylaluminiumhydrid in Tetrahydrofuran in einem Temperaturbereich von -78°C bis Raumtemperatur durchgeführt. Die Hydroxymethylverbindungen werden im zweiten Schritt [2] nach üblichen Methoden zu den Aldehyden (XIII) oxidiert. Die Oxidation kann beispielsweise mit Pyridiniumchlorochromat, gegebenenfalls in Anwesenheit von Aluminiumoxid, in inerten Lösemitteln wie Chlorkohlenwasserstoffen, vorzugsweise Methylenchlorid, in einem Temperaturbereich von 0°C bis 60°C, bevorzugt bei Raumtemperatur durchgeführt werden, oder aber mit Trifluoressigsäure/Dimethylsulfoxid nach den üblichen Methoden der Swern Oxidation durchgeführt werden. Die Aldehyde (VIIb) werden im dritten Schritt [3] mit Diethyl-2-(cyclohexylamino)-vinylphosphonat in Anwesenheit von Natriumhydrid in inerten Lösemitteln wie Ethern, beipielsweise Diethylether, Tetrahydrofuran oder Dioxan, vorzugsweise in Tetrahydrofuran, in einem Temperaturbereich von -20°C bis +40°C, vorzugsweise von -5°C bis Raumtemperatur zu den Aldehyden (IVb) umgesetzt.

Die hierbei als Ausgangsstoffe eingesetzten Verbindungen der Formel (VIa, b) sind neu. Man erhält sie evtl. in Schema B beispielshaft für die Alkoxy-Dihydropyridine (IXb) gezeigt durch Oxidation von 3,4-Dihydropyridinen. Die Oxidation der Dihydropyridine (IXb) zu den Pyridinen (VIb), in welchen R¹⁴ die oben angegebene Bedeutung hat, kann beispielsweise mit Chromoxid oder Natriumnitrit in Eiseissig in einem Temperaturbereich von -20°C bis +150°C, mit Salpetersäure in wäßriger Suspension oder mit Cersalzen, wie beispielsweise Ammoniumcernitrat, in einem Lösungsmittelgemisch aus Acetonitril und Wasser durchgeführt werden. Vorzugsweise wird mit Ammoniumcernitrat in dem Gemisch Acetonnitril und Wasser umgesetzt. 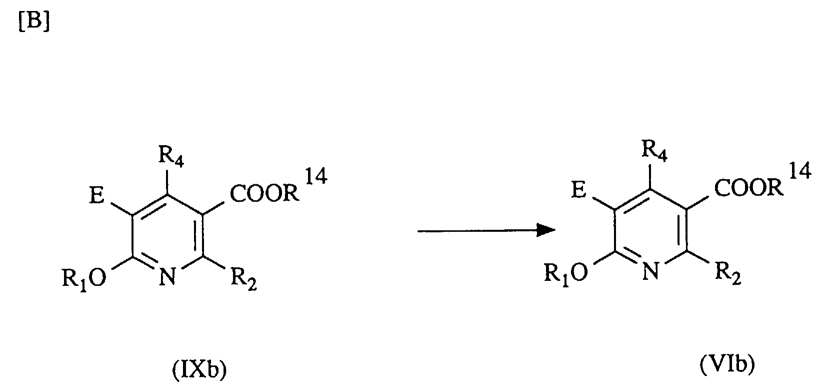 Die hierbei als Ausgangsstoffe eingesetzten 3,4-Dihydropyridine der allgemeinen Formel (IXa,b) sind neu.

Man erhält sie im allgemeinen durch Reaktion geeignet substituierter α,β-ungesättigter Carbonsäureester der allgemeinen Formel (X) und entsprechend substituierten β-Amino-α,β-ungesättigten Carbonsäureestern der allgemeinen Formel (XI) und anschließende Alkylierung.

Das Verfahren kann in Substanz oder in einem hochsiedendem Lösemittel wie beispielsweise Ethylenglykol entweder basisch mit Alkalialkoholaten, wie beispielsweise Natrium- oder Kaliummethanolat bei Raumtemperatur bis +200°C oder in Eisessig bei Raumtemperatur durchgeführt werden. Bevorzugt ist die Umsetzung mit Alkalialkoholaten bei +140°C.

Die Reaktion kann durch folgendes Formelschema erläutert werden: 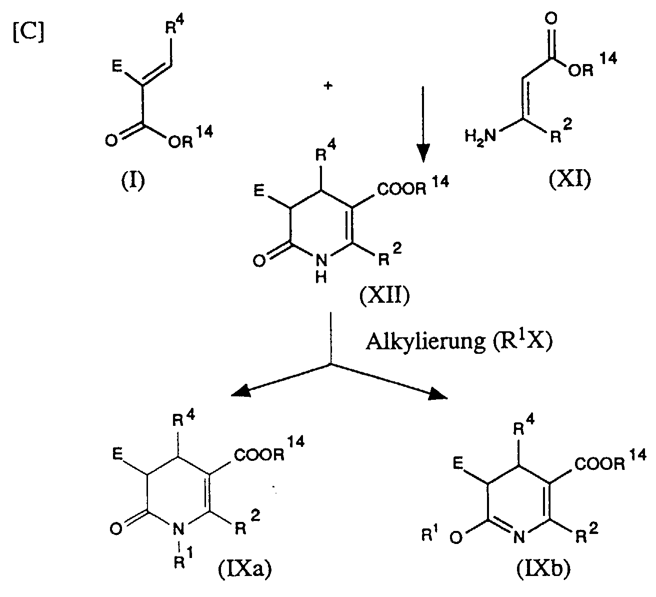 Die Alkylierung erfolgt im allgemeinen mit Alkyl- oder Benzylhalogeniden in Gegenwart einer Base wie beispielsweise Kaliumcarbonat, Natriumhydrid oder einem Säurehalogenid in Gegenwart einer Base wie Imidazol, Pyridin oder Triethylamin können die O-Alkyl bzw. O-Acylderivate hergestellt werden.

Im Fall, daß D und/oder E für den Rest der Formel 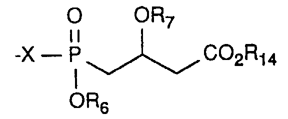 steht, sind die Verbindungen größenteils neu.

Sie können aber in Analogie zu dem oben beschriebenen Verfahren hergestellt werden, indem man ausgehend von den Verbindungen der allgemeinen Formeln 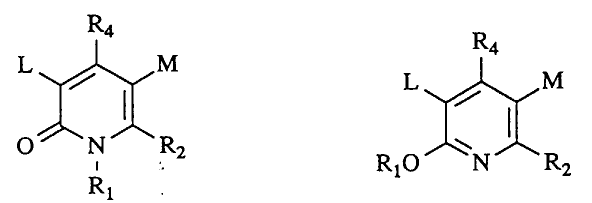 in welcher
R¹, R² und R⁴die oben angegebene Bedeutung haben
L und/oder M für den Rest der Formel 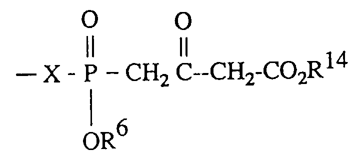 stehen in welcher
-X-, und R⁶ und R¹⁴die angegebene Bedeutung haben
und
R¹⁴ für C₁-C₆-Alkyl steht,
reduziert

Als Lösemittel eignen sich in diesem Fall insbesondere Alkohole wie Methanol, Ethanol oder Propanol, bevorzugt Ethanol.

Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat oder Lithiumaluminiumhydrid durchgeführt. Ganz besonders bevorzugt wird die Reduktion mit Natriumborhydrid, in Anwesenheit von Triethylboran durchgeführt.

Die Reduktion verläuft im allgemeinen in einem Temperaturbereich von -78°C bis +50°C, bevorzugt von -78°C bis +25°C und Normaldruck.

Die Verbindungen der allgemeinen Formel (III) sind größenteils neu, können aber in Analogie zu bekannten Methoden hergestellt werden [vgl. DE 38 172 98 A; US 50 91 378].

Im Fall der enantiomerenreinen Verbindungen der allgemeinen Formel (I) werden entweder die entsprechenden enantiomerenreinen Ester der allgemeinen Formel (II) eingesetzt, die nach publizierten Verfahren durch Umsetzung der racemischen Produkten mit enantiomerenreinen Aminen zu den entsprechenden diastereomeren Amidgemischen, anschließender Trennung durch Chromatographie oder Kristallisation in die einzelnen Diastereomere und abschließender Hydrolyse erhalten werden [vgl. DOS 40 40 026] oder indem man die racemischen Endprodukte nach üblichen chromatographischen Methoden trennt.

Die erfindungsgemäßen substituierten 4-Phenyl-pyridone und 4-Phenyl-2-alkoxy-pyridine sowie deren isomere Formen besitzen wertvolle, im Vergleich zum Stand der Technik überlegene, pharmakologische Eigenschaften, insbesondere sind sie in vivo hochwirksame Inhibitoren der 3-Hydroxy-3-methyl-glutarylCoenzym A (HMG-CoA) Reduktase und infolge dessen Inhibitoren der Cholesterolbiosynthese. Sie können deshalb zur Behandlung von Hyperlipoproteinämie oder Arteriosklerose eingesetzt werden. Die erfindungsgemäßen Wirkstoffe bewirken außerdem eine Senkung des Cholesteringehaltes im Blut.

Die pharmakologische Wirkung der erfindungsgemäßen Stoffe wurde in folgendem Test bestimmt:

### Biologischer Test für HMG-CoA-Reduktaseinhibitoren

Cholesterin wird im Säugetierorganismus aus Acetateinheiten aufgebaut. Um die hepatische Cholesterinbiosynthese in vivo zu messen, wurde den Tieren radioaktiv markiertes ¹⁴C-Acetat appliziert und später der Gehalt an ¹⁴C-Cholesterin in der Leber bestimmt.

Die zu untersuchenden Substanzen wurden auf eine Hemmung der hepatischen Cholesterinbiosynthese in vivo an männlichen Wistar-Ratten mit einem Körpergewicht zwischen 140 und 160 g geprüft. Die Ratten wurden zu diesem Zweck 18 h vor der oralen Applikation der Substanzen gewogen, in Gruppen zu 6 Tieren (Kontrollgruppe ohne Substanzbelastung 8 Tiere) eingeteilt und nüchterngesetzt. Die zu untersuchenden Substanzen wurden direkt vor der Applikation in wäßriger 0,75 %iger Traganthsuspension mit einem Ultra-Turrax suspendiert. Die Applikation der Traganthsuspension (Kontrolltiere) bzw. der in Traganth suspendierten Substanzen erfolgte mittels einer Schlundsonde. 2 h nach der oralen Substanzgabe wurde den Tieren ¹⁴C-Acetat (12,5 µCi/Tier) intraperitoneal injiziert.

Weitere 2 h später (4 h nach Substanzapplikation) wurden die Tiere durch Halsschnitt getötet und entblutet. Anschließend wurde der Bauchraum geöffnet und eine Leberprobe von ca. 700 mg zur Bestimmung des aus ¹⁴C-Acetat synthetisierten ¹⁴C-Cholesterins entnommen. Die Extraktion des Cholesterins erfolgte modifiziert nach Duncan et al. (J. Chromatogr. 162(1979)281-292). Die Leberprobe wurde in einem Glaspotter in Isopropanol homogenisiert Nach Schütteln und anschließender Zentrifugation wurde der Überstand mit alkoholischer KOH versetzt und die Cholesterinester verseift. Nach der Verseifung wurde das Gesamtcholesterin mit Heptan ausgeschüttelt und der Überstand eingedampft. Der Rückstand wurde in Isopropanol aufgenommen, in Szintillationsröhrchen überführt und mit LSC-Cocktail aufgefüllt. Das aus ¹⁴C-Acetat in der Leber synthetisierte ¹⁴C-Cholesterin wurde im Flüssigkeitsszintillationszähler gemessen. Der hepatische ¹⁴C-Cholesteringehalt, der nur mit Traganth behandelten Tiere diente als Kontrolle. Die inhibitorische Aktivität der Substanzen wird in % des synthetisierten hepatischen ¹⁴C-Cholesteringehaltes der Traganth-Kontrolltiere (= 100 %) angegeben.

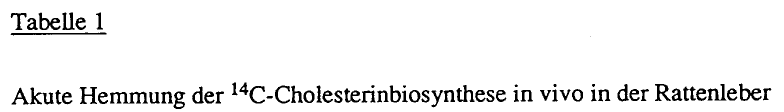

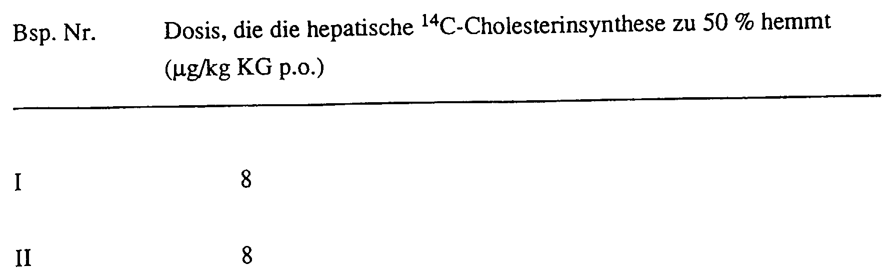

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,1 µg/kg bis etwa 100 µg/kg, bevorzugt in Gesamtmengen von etwa 1 µg/kg bis 50 µg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und dem Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel 1

3-Amino-4-methyl-pent-2-en-säure-ethylester

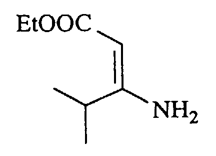

Zu 500 g (3,16 mol) Isobutyrylessigsäureethylester in 1500 ml Toluol p.A. werden 10,8 g p-Toluolsulfonsäure x 4 H₂O gegeben, die Mischung unter Rühren bei Raumtemperatur mit Ammoniak-Gas gesättigt und über Nacht stehen gelassen. Anschließend wird am Wasserabscheider unter Rückfluß erhitzt und kontinuierlich Ammoniak-Gas eingeleitet, bis die berechnete Wassermenge abgeschieden ist (nach 8 Stunden Rückfluß 47 ml Wasser). Man läßt über Nacht abkühlen, saugt den ausgefallenen Niederschlag ab und wäscht mit Toluol nach. Die vereinigten Toluolphasen werden mehrfach mit Wasser gewaschen, mit Natriumsulfat getrocknet, im Vakuum eingeengt und im Hochvakuum destilliert.
Kp.: 82-85°C/1 Torr,
Ausbeute: 315 g (63,4 % der Theorie, ca. 90 %ig).

### Beispiel 2

1-Carbomethoxy-2-(4-fluorphenyl)-propensäure-methylester 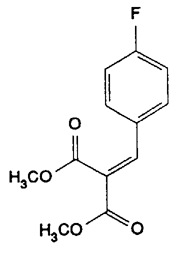 229 ml (2 mol) Malonsäuredimethylester, 223 ml (2 mol) 4-Fluorbenzaldehyd, 40 ml Piperidin und 103 ml Eisessig werden in 1,5 l Cyclohexan über Nacht am Wasserabscheider unter Rückfluß erhitzt. Nach Abkühlen auf Raumtemperatur wird in Essigester aufgenommen, mit Wasser gewaschen, mit Natriumsulfat getrocknet und destilliert.
Sdp.: 135°C - 140°C (1 mm)
Ausbeute: 342,9 g (72 % der Theorie)

### Beispiel 3

3,4-Dihydro-4-(4-fluorphenyl)-6-isopropyl-(1H)-pyrid-2-on-3,5-dicarbonsäure-3-methyl-5-ethylester 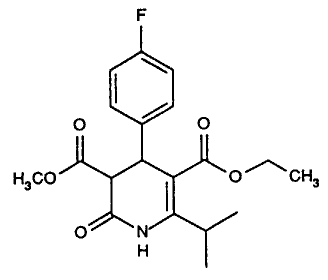 114,3 g (0,48 mol) 1-Carbo-methoxy-2-(4-fluorphenyl)-propensäure-methylester, 75,4 g (0,48 mol) 3-Amino-4-methyl-pent-2en-säureethylester, 1 g Natriummethylat und 5 ml Ethanol wurden 60 h bei 140°C Badtemperatur gerührt und aus Ethanol umkristallisiert.
Schmp.: 124°C
Ausbeute: 115,4 g (66 % der Theorie)

### Beispiel 4

4-(4-Fluorphenyl)-6-isopropyl-(1H)-pyrid-2-on-3,5-dicarbonsäure-3-methyl-5-ethylester 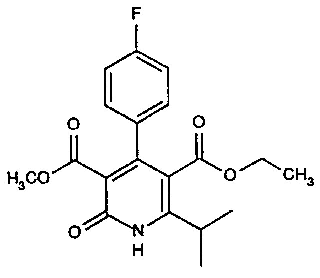 10,8 g (30 mmol) der Verbindung aus Beispiel 3 und 3,9 g (39 mmol) Chromtrioxid wurden in 100 ml Eisessig unter Rückfluß erhitzt, nach 2 h wurden nochmals 2 g (20 mmol) Chromtrioxid zugegeben und über Nacht unter Rückfluß erhitzt. Das Lösemittel wurde abdestilliert, der Rückstand in verdünnter Salzsäure gelöst, mit Ether gewaschen, die vereinigten Etherphasen mit Wasser, wässriger Natriumhydrogencarbonatlösung und Wasser gewaschen, mit Natriumsulfat getrocknet und über Kieselgel 70-230 mesh mit Essigester/Petrolether 1:1 chromatographiert.
Ausbeute: 5,5 g (51 % der Theorie)
Schmelzpunkt: 178°C

### Beispiel 5

4-(4-Fluorphenyl)-6-isopropyl-2-methoxy-pyridin-3,5-dicarbonsäure-3-methyl-5-ethylester 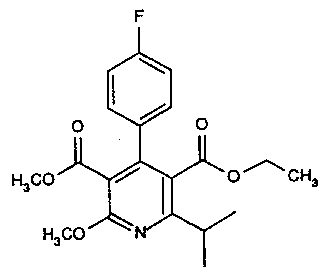 11,3 g (31 mmol) der Verbindung aus Beispiel 4, 1,2 g (50 mmol) Natriumhydrid und 4 ml (62 mmol) Methyliodid werden in 50 ml Dimethylformamid 2 h bei 80°C erhitzt, die Mischung wird bei Raumtemperatur auf 500 ml Wasser gegossen und dreimal mit 150 ml Ether extrahiert. Die vereinigten organischen Phasen werden mit Wasser gewaschen und mit Natriumsulfat getrocknet. Nach Abdestillieren des Lösemittels im Vakuum erhält man 11,1 g.
Rohausbeute: 95,2 % der Theorie
Schmelzpunkt: 83°C

### Beispiel 6

4-(4-Fluorphenyl)-3-hydroxymethyl-6-isopropyl-2-methoxy-pyddin-5-carbonsäureethylester 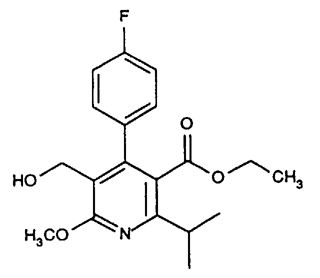 1,48 g (3,95 mmol) der Verbindung aus Beispiel 5 werden in 30 ml Toluol gelöst, unter Stickstoff auf -78°C gekühlt und bei dieser Temperatur 6,6 ml (10 mmol) einer 1,5 molaren Lösung von Diisobutylaluminiumhydrid in Toluol zugetropft. Man entfernt das Kühlbad und rührt 2 h bei Raumtemperatur nach. Nach Hydrolyse mit 20 %iger wässriger Kalium-Natriumtartratlösung wird die organische Phase abgetrennt, die wässrige Phase dreimal mit Toluol gewaschen, die vereinigten organischen Phasen mit gesättigter Natriumchloridlösung gewaschen und mit Natriumsulfat getrocknet.
Nach Abdestillieren des Lösemittels im Vakuum erhält man 1,52 g Öl, das über Kieselgel (Essigester/Petrolether 1:5) chromatographiert wird.
Ausbeute: 520 mg (38 % der Theorie) und 310 mg (21 %) Edukt

### Beispiel 7

4-(4-Fluorphenyl)-6-isopropyl-3-methoxymethyl-2-methoxy-pyridin-5-carbonsäureethylester 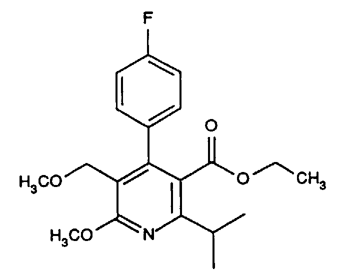 520 mg (1,5 mmol) der Verbindung aus Beispiel 6 werden in 42 mg (1,75 mol) Natriumhydrid und 0,3 ml (4,5 mmol) Methyliodid in 4 ml Dimethylformamid 4 h bei Raumtemepratur gerührt. Das Reaktionsgemisch wird auf Eiswasser gegossen, dreimal mit Ether gewaschen, die vereinigten Etherphasen mit Wasser und gesättigter Natriumchloridlösung gewaschen und mit Natriumsulfat getrocknet. Nach Entfernen des Lösemittels am Rotationsverdampfer erhält man 520 mg Öl.
Ausbeute: 100 % der Theorie

### Beispiel 8

4-(4-Fluorphenyl)-5-hydroxymethyl-6-isopropyl-2-methoxy-3-methoxy-methyl-pyridin 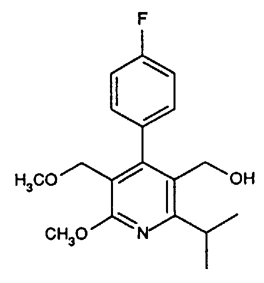 1,19 g (3,5 mmol) der Verbindung aus Beispiel 7 wurden analog Beispiel 6 mit 5,2 ml (7,7 mmol) einer 1,5 molaren Lösung von Diisobutylaluminiuinhydrid in Toluol reduziert. Nach Chromatographie über Kieselgel (Essigester/Petrolether 1:5) erhält man 730 mg Feststoff.
Ausbeute: 66 % der Theorie
Schmlezpunkt:

### Beispiel 9

4-(4-Fluorphenyl)-6-isopropyl-2-methoxy-3-methoxymethyl-pyridin-5-carbaldehyd 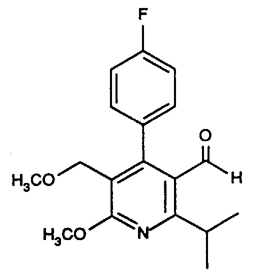 Zu einer Lösung von 0,7 g (2,2 mmol) der Verbindung aus Beispiel 8 in 120 ml Methylenchlorid gibt man 568 mg (2,64 mmol) Pyridiniumchlorochromat, rührt über Nacht bei Raumtemperatur, saugt über Kieselgur ab, wäscht mit 200 ml Methylenchlorid nach, saugt über Kieselgel ab, wäscht mit 200 ml Methylenchlorid nach, trocknet mit Natriumsulfat und erhält nach Entfernen des Lösemittels am Rotationsverdampfer
670 mg Öl.
Ausbeute: 96 % der Theorie

### Beispiel 10

### (E)-3-[4-(4-Fluorphenyl)-6-isopropyl-2-methoxy-3-methoxymethyl-pyridin-5-yl]-prop-2-enal

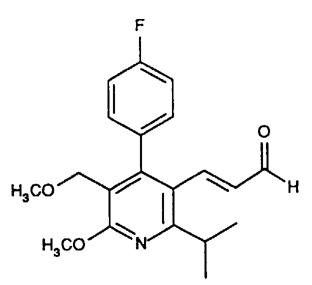

Unter Stickstoff tropft man zu einer Suspension von 59 mg (2,5 mmol) Natriumhydrid in 6 ml trockenem Tetrahydrofuran bei -5°C 804 mg (3,1 mmol) Diethyl-2-(cyclohexylamino)-vinylphosphonat gelöst in 6 ml trockenem Tetrahydrofuran. Nach 30 min. werden bei derselben Temperatur 0,65 g (2,05 mmol) der Verbindung aus Beispiel 9 in 15 ml trockenem Tetrahydrofuran zugetropft und 30 min zum Rückfluß erwärmt. Nach Abkühlen auf Raumtemperatur wird der Ansatz in 200 ml eiskaltes Wasser gegeben und dreimal mit je 100 ml Essigester extrahiert Die vereinigten organischen Phasen werden mit gesättigter Natriumchlorid-Lösung gewaschen und über Natriumsulfat getrocknet. Nach Einengen im Vakuum wird der Rückstand in 5 ml Toluol aufgenommen, mit einer Lösung von 0,9 g (7 mmol) Oxalsäure-Dihydrat in 12 ml Wasser versetzt und 90 min zum Rückfluß erhitzt. Nach Abkühlen auf Raumtemperatur werden die Phasen getrennt, die organische Phase mit gesättigter Natriumchlorid-Lösung gewaschen, über Natriumsulfat getrocknet und im Vakuum eineengt. Der Rückstand wird in Methylenchlorid gelöst und über Kieselgel filtriert.
Ausbeute: 560 mg Feststoff (79,6 % der Theorie)

### Beispiel 11

Methyl-(E)-7-[4-(4-fluorphenyl)-6-isopropyl-2-methoxy-3-methoxy-methyl-pyridin-5-yl]-5-hydroxy-3-oxo-hept-6-enoat 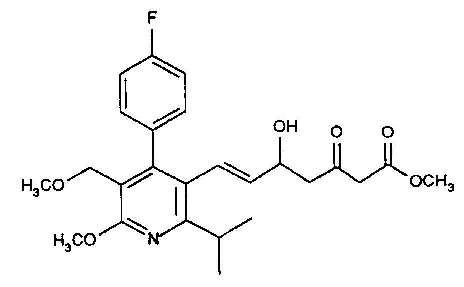 Unter Stickstof tropft man zu einer Suspension von 80 mg (3,4 mmol) Natriumhydrid in 3 ml trockenem Tetrahydrofuran bei -5°C 0,35 ml (3,3 mmol) Acetessigsäuremethylester. Nach 15 min werden bei derselben Temperatur 2,3 ml (3,3 mmol) 15 %iges Butyllithium in n-Hexan und 3,3 ml (3,3 mmol) einer 1 molaren Zinkchlorid-Lösung in Ether zugetropft und 15 min nachgerührt Anschließend werden 530 mg (1,5 mmol) der Verbindung aus Beispiel 10 gelöst in 8 ml trockenem Tetrahydrofuran zugetropft und 30 min bei -5°C nachgerührt. Die Reaktionslösung wird vorsichtig mit 100 ml gesättigter wässriger Ammoniumchloridlösung verdünnt und die Mischung dreimal mit je 100 ml Ether extrahiert. Die vereinigten organischen Phasen werden zweimal mit gesättigter Natriumhydrogencarbonat-Lösung und einmal mit gesättigter Natriumchlorid-Lösung gewaschen, über Natriumsulfat getrocknet und im Vakkum eingeengt.
Rohausbeute: 760 mg (100 % der Theorie)

### Beispiel 12

Methyl-erythro-(E)-7-[4-(4-fluorphenyl)-6-isopropyl-2-methoxy-3-methoxymethylpyrid-5-yl]-3,5-dihydroxy-hept-6-enoat 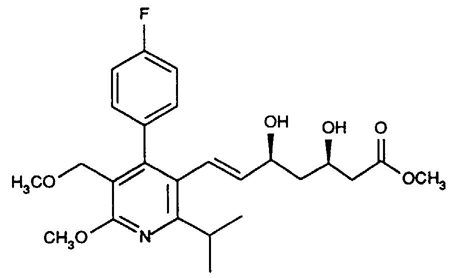 Zu einer Lösung von 730 mg (1,6 mmol) der Verbindung aus Beispiel 11 in 13 ml trockenem Tetrahydrofuran gibt man bei Raumtemperatur 1,9 ml (1,9 mmol) 1 M Triethylboran-Lösung in Tetrahydrofuran, leitet während 5 min Luft durch die Lösung und kühlt auf -30°C Innentemperatur ab. Es werden 72 mg (1,9 mmol) Natriumborhydrid und langsam 1,3 ml Methanol zugegeben, 30 min bei -30°C gerührt und dann mit einem Gemisch von 5 ml 30 %igem Wasserstoffperoxid und 11 ml Wasser versetzt. Die Temperatur läßt man dabei bis 0°C ansteigen und rührt noch 30 min nach. Die Mischung wird dreimal mit je 70 ml Essigester extrahiert, die vereinigten organischen Phasen werden je einmal mit 10 %iger Kaliumiodidlösung, 10 %iger Natriumthiosulfatlösung, gesättigter Natriumhydrogencarbonat-Lösung und gesättigter Natriumchlorid-Lösung gewaschen, über Natriumsulfat getrocknet und im Vakkum eingeengt. Der Rückstand wird an einer Säule (100 g Kieselgel 230 - 400 mesh, Essigester/Petrolether 1:2) chromatographiert.
Ausbeute: 350 mg Öl (47,6 % der Theorie)

### Beispiel 13

3-(tert.-Butyldimethylsilyloxymethyl)-4-(4-fluorphenyl)-6-isopropyl-2-methoxypyridin-5-carbonsäureethylester

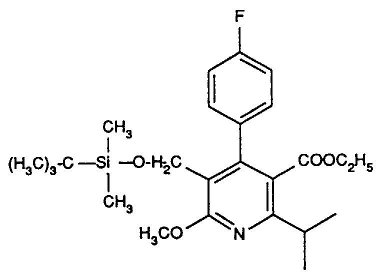

Zu einer Lösung von 600 mg (1,8 mmol) der Verbindung aus Beispiel 6 in 20 ml Dimethylformamid gibt man bei Raumtemperatur 304 mg (2 mmol) tert.-Butyldimethylsilylchlorid, 262 mg (4 mmol) Imidazol und 0,05 g 4-Dimethylaminopyridin. Es wird über Nacht bei Raumtemepratur gerührt, mit 200 ml Wasser versetzt und mit 1 N Salzsäure auf pH 3 eingestellt. Die Mischung wird dreimal mit je 100 ml Ether extrahiert, die vereinigten organischen Phasen einmal mit gesättigter Natriumchlorid-Lösung gewaschen, über Magnesiumsulfat getrocknet und im Vakuum eingeengt. Der Rückstand wird an einer Säule (150 g Kieselgel, 70-230 mesh, ⌀ 4 cm, mit Essigester/Petrolether 1:9) chromatographiert.
Ausbeute: 700 mg (87 % der Theorie)

### Beispiel 14

Methyl-erythro-(E)-7-[3-tert.-butyldimethylsilyloxy-methyl-4-(4-fluorphenyl)-6-isopropyl-2-methoxy-pyridin-5-yl]-3,5-dihydroxy-hept-6-enoat 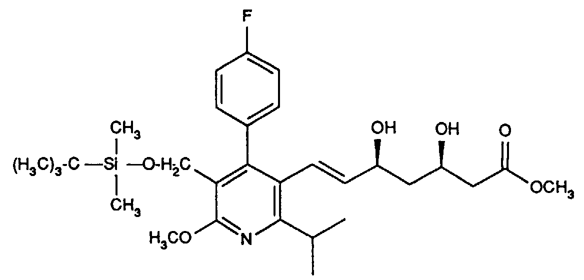 Ausgehend von Beispiel 13 wurde analog zu den Vorschriften der Beispiele 8-12 die Titelverbindung hergestellt.

### Beispiel 15

Methyl-erythro-(E)-7-[4-(4-fluorphenyl)-3-hydroxymethyl-6-isopropyl-2-methoxy-pyridin-5-yl]-5-dihydroxy-hept-6-enoat 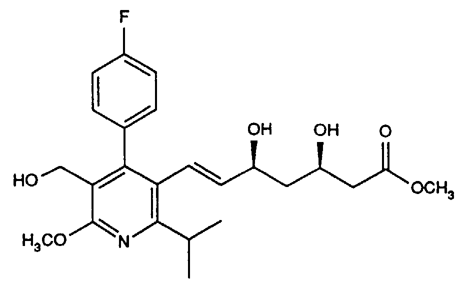 100 mg (0,18 mmol) der Verbindung aus Beispiel 14 werden in einer Lösung von 1 ml 1 N-Salzsäure und 9 ml Methanol über Nacht bei Raumtemperatur gerührt. Nach dem Einengen wird mit Methylenchlorid aufgenommen, mit gesättigter Natriumhydrogencarbonatlösung gewaschen, getrocknet und über Kieselgel filtriert (Essigester/Petrolether 1:1).
Ausbeute: 46 mg (57 % der Theorie)
Schmelzpunkt:

### Herstellbeispiele

### Beispiel I

3S,5S-(+)-(E)-7-[4-(4-fluorphenyl)-6-isopropyl-2-methoxy-3-methoxymethyl-pyrid-5-yl]-hept-6-en-1,3,5-triol 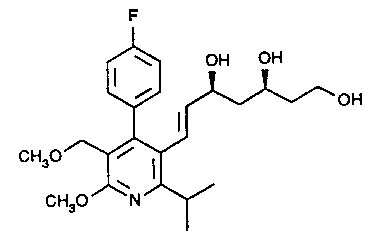 3,92 g (8,5 mmol) 3R,5S-(+)-Methyl-(E)-7-[4-(4-fluorphenyl)-6-isopropyl-2-methoxy-3-methoxymethyl-pyrid-5-yl]-3,5-dihydroxy-hept-6-enoat werden unter Argon in 100 ml absolutem THF gelöst Bei -78°C werden 35,8 ml (43 mmol) einer 1,2 M Diisobutylaluminiumhydrid-Lösung (in Toluol) zugetropft. Nach 12 h bei -30°C läßt man die Reaktionslösung auf 0°C kommen und gibt vorsichtig 150 ml Wasser zu. Anschließend wird 3 mal mit je 200 ml Essigester extrahiert Die organische Phase wird mit gesättigter NaCl-Lösung gewaschen, mit Na₂SO₄ getrocknet und am Rotationsverdampfer eingeengt. Nach Chromatographie über Kieselgel 60 (25 - 40 µ, Laufmittel Essigester / Petrolether 7/3) erhält man das gewünschte Produkt.
Ausbeute: 1,54 g (42% d.Th.)
¹H-NMR (CDCl₃): δ = 1,22 (m, 6H); 1,43 (m, 2H); 1,62 (m, 2H); 3,22 (s, 3H); 3,25 (sept. 1H); 3,84 (m, 2H); 4,01 (s, 3H); 4,04 (m, 1H); 4,06 (s; 2H); 4,28 (m, 1H); 5,22 (dd, 1H); 6,25 (d, 1H); 7,0 - 7,2 (m, 4H) ppm.

### Beispiel II

3S,5S-(+)-(E)-7-[4-(4-fluorphenyl)-3-hydroxymethyl-6-isopropyl-2-methoxy-pyrid-5-yl]-hept-6-en-1,3,5-triol 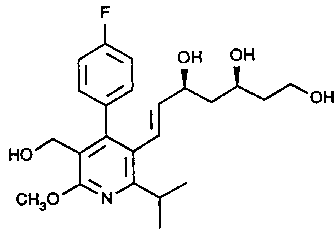 Die Herstellung erfolgt analog Beispiel I aus 3R,5S-(+)-Methyl-(E)-7-[4-(4-fluorphenyl)-3-hydroxymethyl-6-isopropyl-2-methoxy-pyrid-5-yl]-3,5-dihydroxy-hept-6-enoat.
¹H-NMR (CDCl₃): δ = 1,25 (d, 6H); 1,42 (m, 2H); 1,63 (m, 2H); 3,28 (sept. 1H); 3,82 (m, 2H); 4,01 (m, 1H); 4,06 (s, 3H); 4,27 (m, 1H); 4,35 (d, 2H); 5,23 (dd, 1H); 6,22 (d, 1H); 7,0 - 7,2 (m, 4H) ppm.

## Patentansprüche

1. Substituierte 4-Phenyl-pyridone und 4-Phenyl-2-alkoxypyridine der allgemeinen Formel 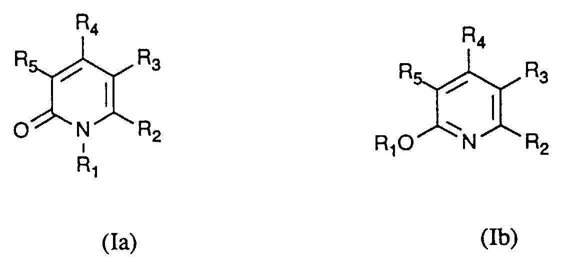 in welcher
R¹ für geradkettiges oder verzweigtes Alkyl mit bis zu 8 Kohlenstoffatomen steht,
R² für Cycloalkyl mit 3 bis 7 Kohlenstoffatomen steht, oder für geradkettiges oder verzweigtes Alkyl mit bis zu 8 Kohlenstoffatomen steht,
R³ für einen Rest der Formel 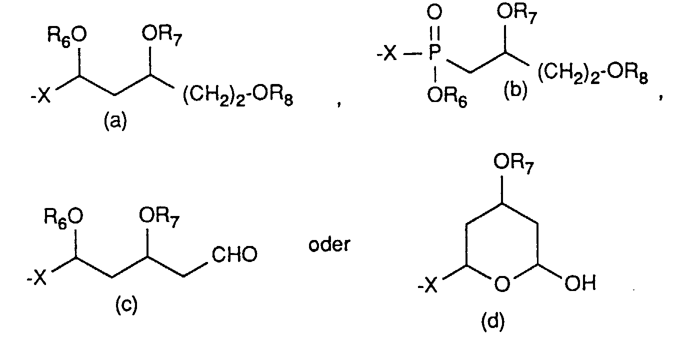 steht,
worin
X die Gruppe -CH₂-CH₂-, -CH=CH- oder -C≡C- bedeutet,
und
R⁶, R⁷ und R⁸ gleich oder verschieden sind und Wasserstoff oder einen Rest der Formel -CO-R⁹ oder -COOR¹⁰ bedeuten,
worin
R⁹ und R¹⁰ gleich oder verschieden sind und geradkettiges oder verzweigtes Alkyl mit bis zu 8 Kohlenstoffatomen oder Phenyl bedeuten,
oder
R⁶ und R⁷ gemeinsam einen Rest der Formel 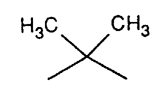 bilden,
R⁴ für Phenyl steht, das gegebenenfalls bis zu 2-fach gleich oder verschieden durch Halogen, Trifluormethyl, Methoxy, Phenoxy oder geradkettiges oder verzweigtes Alkyl mit bis zu 8 Kohlenstoffatomen substituiert ist,
R⁵ die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 8 Kohlenstoffatomen steht, oder
für geradkettiges oder verzweigtes Alkyl mit bis zu 8 Kohlenstoffatomen steht, das gegebenenfalls durch Halogen, Hydroxy, durch geradkettiges oder verzweigtes Alkoxy mit bis zu 8 Kohlenstoffatomen, oder durch eine Gruppe der Formel -O-(CH₂)ₐ-R¹¹ oder -O-CO-R¹² substituiert ist,
worin
a eine Zahl 0 oder 1 bedeutet,
R¹¹ Phenyl oder Benzyl bedeutet, die gegebenenfalls in Aromaten bis zu 2-fach gleich oder verschieden durch Halogen, Trifluormethyl, Cyano, Nitro oder durch geradkettiges oder verzweigtes Alkyl mit bis zu 6 Kohlenstoffatomen substituiert sind,
R¹² die oben angegebene Bedeutung von R¹¹ hat und mit dieser gleich oder verschieden ist, oder
geradkettiges oder verzweigtes Alkly mit bis zu 8 Kohlenstoffatomen bedeutet,
oder
R⁵ für einen Rest der Formel -CH=N-O-R¹³ steht,
worin
R¹³ die oben angegebene Bedeutung von R¹² hat und mit dieser gleich oder verschieden ist

2. Substituierte 4-Phenyl-pyridone und 4-Phenyl-2-alkoxypyridine nach Anspruch 1,
wobei
R¹ für geradkettiges oder verzweigtes Alkyl mit bis zu 6 Kohlenstoffatomen steht,
R² für Cyclopropyl, Cyclopentyl oder Cyclohexyl steht, oder für geradkettiges oder verzweigtes Alkyl mit bis zu 6 Kohlenstoffatomen steht,
R³ für einen Rest der Formel 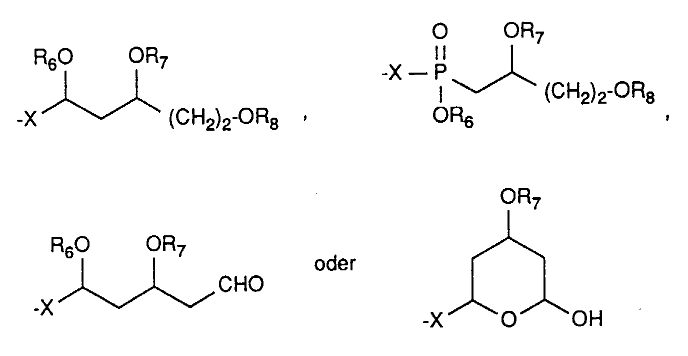 steht,
worin
X die Gruppe -CH₂-CH₂-, -CH=CH- oder -C≡C- bedeutet,
R⁶, R⁷ und R⁸ gleich oder verschieden sind und Wasserstoff oder einen Rest der Formel -CO-R⁹ oder -COO-R¹⁰ bedeuten,
worin
R⁹ und R¹⁰ gleich oder verschieden sind und geradkettiges oder verzweigtes Alkyl mit bis zu 6 Kohlenstoffatomen oder Phenyl bedeuten,
oder
R⁶ und R⁷ gemeinsam einen Rest der Formel 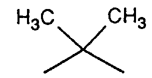 bilden,
R⁴ für Phenyl steht, das gegebenenfalls bis zu 2-fach gleich oder verschieden durch Fluor, Chlor, Brom, Trifluormethyl oder durch geradkettiges oder verzweigtes Alkyl mit bis zu 6 Kohlenstoffatomen substituiert ist,
R⁵ die oben angegebene Bedeutung von R³ hat und mit dieser gleich oder verschieden ist, oder
für Wasserstoff, Cyano, Carboxy oder geradkettiges oder verzweigtes Alkoxycarbonyl mit bis zu 6 Kohlenstoffatomen steht, oder
für geradkettiges oder verzweigtes Alkyl mit bis zu 6 Kohlenstoffatomen steht, das gegebenenfalls durch Fluor, Chlor, Brom, Hydroxy oder durch geradkettiges oder verzweigtes Alkoxy mit bis zu 6 Kohlenstoffatomen substituiert ist, oder das durch eine Gruppe der Formel -O-(CH₂)ₐ-R¹¹ oder -O-CO-R¹² substituiert ist,
worin
a eine Zahl 0 oder 1 bedeutet,
und
R¹¹ Phenyl oder Benzyl bedeutet, die in Aromaten gegebenenfalls durch Fluor, Chlor, Brom, Trifluormethyl, Cyano, Nitro oder durch geradkettiges oder verzweigtes Alkyl mit bis zu 4 Kohlenstoffatomen substituiert sind,
R¹² die oben angegebene Bedeutung von R¹¹ hat und mit dieser gleich oder verschieden ist, oder
geradkettiges oder verzweigtes Alkyl mit bis zu 6 Kohlenstoffatomen bedeutet,
oder
R⁵ für einen Rest der Formel -CH=N-O-R¹³ steht,
worin
R¹³ die oben angegebene Bedeutung von R¹² hat und mit dieser gleich oder verschieden ist.

3. Substituierte 4-Phenyl-pyridone und 4-Phenyl-2-alkoxypyridine nach Anspruch 1
wobei
R¹ für geradkettiges oder verzweigtes Alkyl mit bis zu 4 Kohlenstoffatomen steht,
R² für Cyclopropyl oder für geradkettiges oder verzweigtes Alkyl mit bis zu 4 Kohlenstoffatomen steht,
R³ für einen Rest der Formel 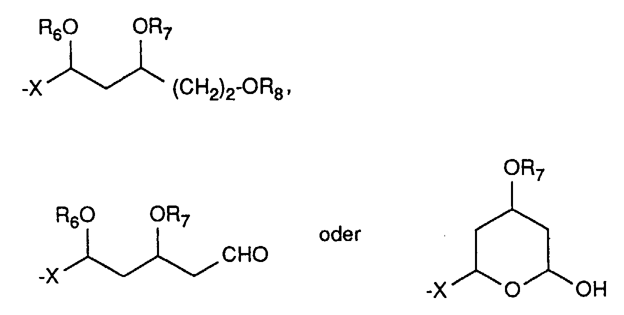 steht,
worin
X die Gruppe -CH₂-CH₂- oder -CH=CH- bedeutet,
R⁶, R⁷ und R⁸ gleich oder verschieden sind und Wasserstoff oder einen Rest der Formel -CO-R⁹ bedeuten,
worin
R⁹ geradkettiges oder verzweigtes Alkyl mit bis zu 4 Kohlenstoffatomen bedeutet,
R⁴ für Phenyl steht, das gegebenenfalls durch Fluor, Trifluormethyl oder durch geradkettiges oder verzweigtes Alkyl mit bis zu 4 Kohlenstoffatomen substituiert ist,
R⁵ die oben angegebene Bedeutung von R³ hat und mit dieser gleich oder verschieden ist, oder
für Wasserstoff oder
für geradkettiges oder verzweigtes Alkyl mit bis zu 4 Kohlenstoffatomen steht, das gegebenenfalls durch Hydroxy oder durch geradkettiges oder verzweigtes Alkoxy mit bis zu 4 Kohlenstoffatomen substituiert ist.

4. Substituierte 4-Phenyl-pyridone und 4-Phenyl-2-alkoxypyridine nach Anspruch 1
wobei
R¹ für geradkettiges oder verzweigtes Alkyl mit bis zu 4 Kohlenstoffatomen steht,
R² für Cyclopropyl oder für geradkettiges oder verzweigtes Alkyl mit bis zu 4 Kohlenstoffatomen steht,
R³ für einen Rest der Formel 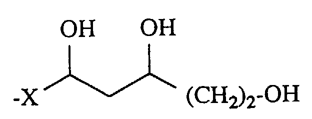 steht,
wobei
X die Gruppe -CH₂-CH₂- oder -CH=CH- bedeutet,
R⁴ für Phenyl steht, das gegebenenfalls durch Fluor substituiert ist, und
R⁵ die oben angegebene Bedeutung von R³ hat oder
für gradkettiges oder verzweigtes Alkyl mit bis zu 4 Kohlenstoffatomen steht, das gegebenenfalls durch Hydroxy oder Alkoxy mit bis zu vier Kohlenstoffatomen substituiert ist.

5. Substituierte 4-Phenyl-pyridone und 4-Phenyl-2-alkoxypyridine nach Anspruch 1 zur therapeutischen Anwendung.

6. Verfahren zur Herstellung von substituierten 4-Phenyl-pyridonen und 4-Phenyl-2-alkoxypyridinen nach Anspruch 1, dadurch gekennzeichnet, daß man Pyridone der allgemeinen Formel II 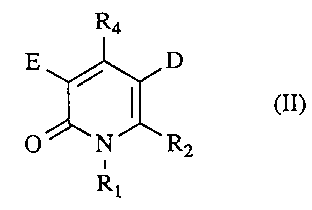 in welcher
D für einen Rest der Formel 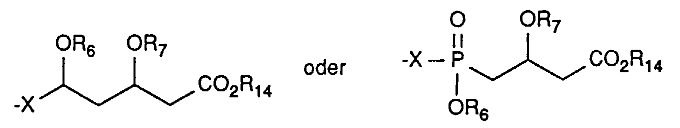 steht,
worin
R¹⁴ für C₁-C₆-Alkyl steht
in inerten Lösemitteln, unter Schutzgasatmosphäre, gegebenenfalls über die Stufe des Aldehyds, mit Reduktionsmitteln reduziert,
und im Fall, daß -X- für die -CH₂-CH₂-Gruppe steht, die Ethengruppe (X = -CH=CH-) oder die Ethingruppe (X = -C≡C-) schrittweise nach üblichen Methoden hydriert, und gegebenenfalls die Isomeren trennt.

7. Arzneimittel enthaltend mindestens ein substituiertes 4-Phenyl-pyridon oder 4-Phenyl-2-alkoxypyridin nach Anspruch 1.

8. Arzneimittel nach Anspruch 1 zur Behandlung von Hyperlipoproteinämie.

9. Verfahren zur Herstellung von Arzneimittel nach Anspruch 7 dadurch gekennzeichnet, daß man die substituierten 4-Phenyl-pyridone oder 4-Phenyl-2-alkoxypyridine gegebenenfalls mit Hilfe von üblichen Hilfs- und Trägerstoffen in eine geeignete Applikationsform überführt.

10. Verwendung von substituierten 4-Phenyl-pyridonen und 4-Phenyl-2-alkoxypyridinen zur Herstellung von Arzneimitteln.

## Claims

1. Substituted 4-phenyl-pyridones and 4-phenyl-2-alkoxypyridines of the general formula 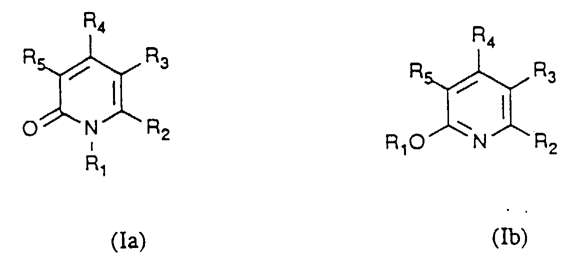 in which
R¹ represents straight-chain or branched alkyl having up to 8 carbon atoms,
R² represents cycloalkyl having 3 to 7 carbon atoms, or
represents straight-chain or branched alkyl having up to 8 carbon atoms,
R³ represents a radical of the formula 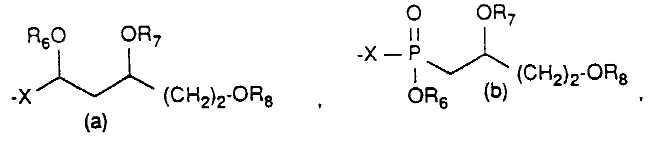 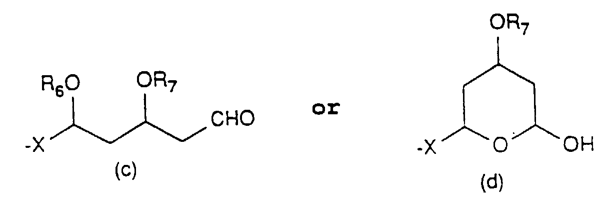
in which
X denotes the group -CH₂-CH₂-, -CH=CH- or -C≡C-,
and
R⁶, R⁷ and R⁸ are identical or different and denote hydrogen or a radical of the formula -CO-R⁹ or -COOR¹⁰,
in which
R⁹ and R¹⁰ are identical or different and denote straight-chain or branched alkyl having up to 8 carbon atoms, or phenyl,
or
R⁶ and R⁷ together form a radical of the formula 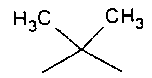
R⁴ represents phenyl which is optionally substituted up to 2 times by identical or different halogen, trifluoromethyl, methoxy, phenoxy or straight-chain or branched alkyl having up to 8 carbon atoms,
R⁵ has the abovementioned meaning of R³ and is identical to or different from this, or
represents hydrogen, cyano, carboxyl or straight-chain or branched alkoxycarbonyl having up to 8 carbon atoms, or
represents straight-chain or branched alkyl having up to 8 carbon atoms, which is optionally substituted by halogen, hydroxyl, by straight-chain or branched alkoxy having up to 8 carbon atoms, or by a group of the formula -O-(CH₂)ₐ-R¹¹ or -O-CO-R¹²,
in which
a denotes a number 0 or 1,
R¹¹ denotes phenyl or benzyl, each of which is optionally substituted up to 2 times in the aromatic system by identical or different halogen, trifluoromethyl, cyano, nitro or straight-chain or branched alkyl having up to 6 carbon atoms,
R¹² has the abovementioned meaning of R¹¹ and is identical to or different from this, or
denotes straight-chain or branched alkyl having up to 8 carbon atoms,
or
R⁵ represents a radical of the formula -CH=N-O-R¹³,
in which
R¹³ has the abovementioned meaning of R¹² and is identical to or different from this.

2. Substituted 4-phenyl-pyridones and 4-phenyl-2-alkoxypyridines according to Claim 1,
in which
R¹ represents straight-chain or branched alkyl having up to 6 carbon atoms,
R² represents cyclopropyl, cyclopentyl or cyclohexyl, or represents straight-chain or branched alkyl having up to 6 carbon atoms,
R³ represents a radical of the formula 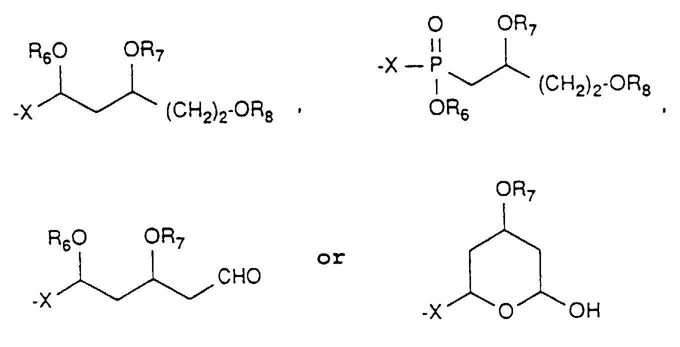 in which
X denotes the group -CH₂-CH₂-, -CH=CH- or -C≡C-,
R⁶, R⁷ and R⁸ are identical or different and denote hydrogen or a radical of the formula -CO-R⁹ or -COO-R¹⁰,
in which
R⁹ and R¹⁰ are identical or different and denote straight-chain or branched alkyl having up to 6 carbon atoms, or phenyl,
or
R⁶ and R⁷ together form a radical of the formula 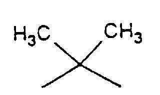
R⁴ represents phenyl which is optionally substituted up to 2 times by identical or different fluorine, chlorine, bromine, trifluoromethyl or straight-chain or branched alkyl having up to 6 carbon atoms,
R⁵ has the abovementioned meaning of R³ and is identical to or different from this, or
represents hydrogen, cyano, carboxyl or straight-chain or branched alkoxycarbonyl having up to 6 carbon atoms, or
represents straight-chain or branched alkyl having up to 6 carbon atoms, which is optionally substituted by fluorine, chlorine, bromine, hydroxyl or by straight-chain or branched alkoxy having up to 6 carbon atoms, or which is substituted by a group of the formula -O-(CH₂)ₐ-R¹¹ or -O-CO-R¹²,
in which
a denotes a number 0 or 1,
and
R¹¹ denotes phenyl or benzyl, each of which is optionally substituted in the aromatic system by fluorine, chlorine, bromine, trifluoromethyl, cyano, nitro or straight-chain or branched alkyl having up to 4 carbon atoms,
R¹² has the abovementioned meaning of R¹¹ and is identical to or different from this, or
denotes straight-chain or branched alkyl having up to 6 carbon atoms,
or
R⁵ represents a radical of the formula -CH=N-O-R¹³,
in which
R¹³ has the abovementioned meaning of R¹² and is identical to or different from this.

3. Substituted 4-phenyl-pyridones and 4-phenyl-2-alkoxypyridines according to Claim 1
in which
R¹ represents straight-chain or branched alkyl having up to 4 carbon atoms,
R² represents cyclopropyl or represents straight-chain or branched alkyl having up to 4 carbon atoms,
R³ represents a radical of the formula 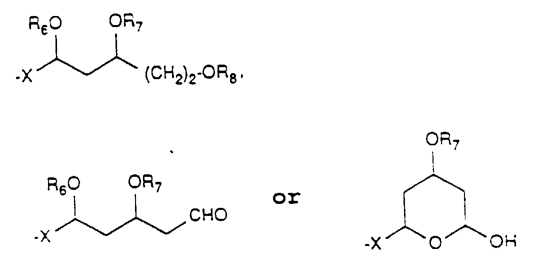
in which
X denotes the group -CH₂-CH₂- or -CH=CH-,
R⁶, R⁷ and R⁸ are identical or different and denote hydrogen or a radical of the formula -CO-R⁹,
in which
R⁹ denotes straight-chain or branched alkyl having up to 4 carbon atoms,
R⁴ represents phenyl which is optionally substituted by fluorine, trifluoromethyl or by straight-chain or branched alkyl having up to 4 carbon atoms,
R⁵ has the abovementioned meaning of R³ and is identical to or different from this, or represents hydrogen or
represents straight-chain or branched alkyl having up to 4 carbon atoms, which is optionally substituted by hydroxyl or by straight-chain or branched alkoxy having up to 4 carbon atoms.

4. Substituted 4-phenyl-pyridones and 4-phenyl-2-alkoxypyridines according to Claim 1
in which
R¹ represents straight-chain or branched alkyl having up to 4 carbon atoms,
R² represents cyclopropyl or represents straight-chain or branched alkyl having up to 4 carbon atoms,
R³ represents a radical of the formula 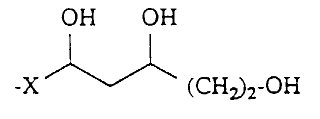 in which
X denotes the group -CH₂-CH₂- or -CH=CH-,
R⁴ represents phenyl which is optionally substituted by fluorine, and
R⁵ has the abovementioned meaning of R³ or represents straight-chain or branched alkyl having up to 4 carbon atoms, which is optionally substituted by hydroxyl or alkoxy having up to four carbon atoms.

5. Substituted 4-phenyl-pyridones and 4-phenyl-2-alkoxypyridines according to Claim 1 for therapeutic use.

6. Process for the preparation of substituted 4-phenylpyridones and 4-phenyl-2-alkoxypyridines according to Claim 1, characterized in that pyridones of the general formula (II) 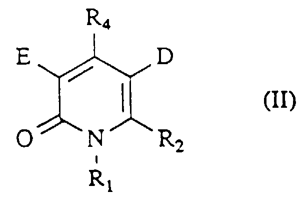 in which
D represents a radical of the formula 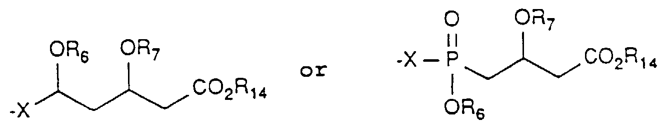 in which
R¹⁴ represents C₁-C₆-alkyl
are reduced in inert solvents, under a protective gas atmosphere, if appropriate via the aldehyde step, using reducing agents,
and in the case in which -X- represents the -CH₂-CH₂- group, the ethene group (X = -CH=CH-) or the ethine group (X = -C≡C-) is hydrogenated stepwise according to customary methods and, if appropriate, the isomers are separated.

7. Medicament containing at least one substituted 4-phenyl-pyridone or 4-phenyl-2-alkoxypyridine according to Claim 1.

8. Medicament according to Claim 1 for the treatment of hyperlipoproteinaemia.

9. Process for the production of medicaments according to Claim 7, characterized in that the substituted 4-phenyl-pyridones or 4-phenyl-2-alkoxypyridines are converted into a suitable administration form, if appropriate with the aid of customary auxiliaries and excipients.

10. Use of substituted 4-phenyl-pyridones and 4-phenyl-2-alkoxypyridines for the production of medicaments.

## Revendications

1. 4-phényl-pyridones et 4-phényl-2-alkoxypyridines substituées de formule générale 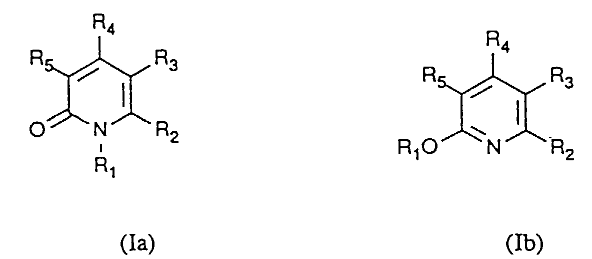 dans laquelle
R¹ est un groupe alkyle linéaire ou ramifié ayant jusqu'à 8 atomes de carbone,
R² est un groupe cycloalkyle de 3 à 7 atomes de carbone ou un groupe alkyle linéaire ou ramifié ayant jusqu'à 8 atomes de carbone,
R³ est un reste de formule 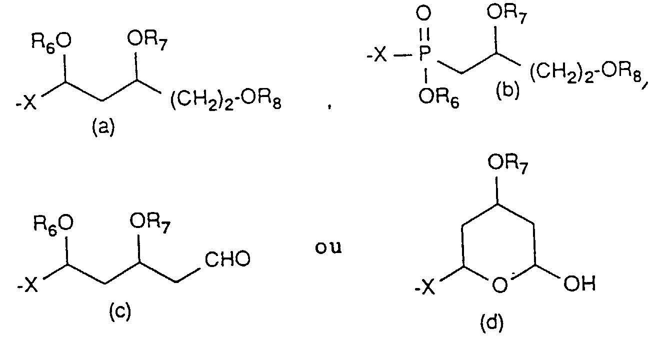
dans laquelle
X représente le groupe -CH₂-CH₂-, -CH=CH- ou -C≡C-,
et
R⁶, R⁷ et R⁸ sont identiques ou différents et représentent de l'hydrogène ou un reste de formule -CO-R⁹ ou -COOR¹⁰,
dans laquelle
R⁹ et R¹⁰ sont identiques ou différents et représentent un groupe alkyle linéaire ou ramifié ayant jusqu'à 8 atomes de carbone ou le groupe phényle,
ou bien
R⁶ et R⁷ forment ensemble un reste de formule 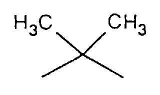
R⁴ est un groupe hényle qui porte, le cas échéant, jusqu'à 2 substituants, identiques ou différents, halogéno, trifluorométhyle, méthoxy, phénoxy ou alkyle linéaire ou ramifié ayant jusqu'à 8 atomes de carbone,
R⁵ a la définition indiquée ci-dessus pour R³ et est identique à R³ ou en est différent, ou représente
de l'hydrogène, un groupe cyano, carboxy ou alkoxycarbonyle linéaire ou ramifié ayant jusqu'8 8 atomes de carbone, ou bien
un groupe alkyle linéaire ou ramifié ayant jusqu'à 8 atomes de carbone, qui est substitué, le cas échéant par un halogène, un radical hydroxy, un radical alkoxy linéaire ou ramifié ayant jusqu'à 8 atomes de carbone ou par un groupe de formule -O-(CH₂)ₐ-R¹¹ ou -O-CO-R¹², dans laquelle
a représente le nombre 0 ou 1,
R¹¹ est un groupe phényle ou un groupe benzyle qui portent, le cas échéant, dans les parties aromatiques, jusqu'à 2 substituants, identiques ou différents, halogéno, trifluorométhyle, cyano, nitro ou bien alkyle linéaire ou ramifié ayant jusqu'à 6 atomes de carbone,
R¹² a la définition indiquée ci-dessus pour R¹¹ et est identique à R¹¹ ou en est différent, ou représente
un groupe alkyle linéaire ou ramifié ayant jusqu'à 8 atomes de carbone,
ou bien
R⁵ est un reste de formule -CH=N-O-R¹³
dans laquelle
R¹³ a la définition indiquée ci-dessus pour R¹² et est identique à R¹² ou en est différent.

2. 4-phényl-pyridones et 4-phényl-2-alkoxypyridines substituées suivant la revendication 1,
dans lesquelles
R¹ est un groupe alkyle linéaire ou ramifié ayant jusqu'à 6 atomes de carbone,
R² est un groupe cyclopropyle, cyclopentyle ou cyclohexyle, ou
un groupe alkyle linéaire ou ramifié ayant jusqu'à 6 atomes de carbone,
R³ est un reste de formule 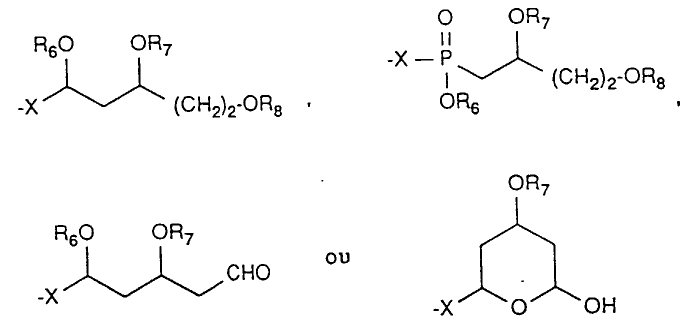
dans laquelle
X représente le groupe -CH₂-CH₂-, -CH=CH- ou -C≡C,
R⁶, R⁷ et R⁸ sont identiques ou différents et représentent de l'hydrogène ou un reste de formule -CO-R⁹ ou -COO-R¹⁰,
dans laquelle
R⁹ et R¹⁰ sont identiques ou différents et représentent un groupe alkyle linéaire ou ramifié ayant jusqu'à 6 atomes de carbone ou le groupe phényle,
ou bien
R⁶ et R⁷ forment ensemble un reste de formule 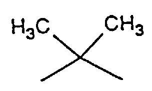
R⁴ est un groupe phényle qui porte, le cas échéant, jusqu'à 2 substituants, identiques ou différents, fluoro, chloro, bromo, trifluorométhyle ou bien alkyle linéaire ou ramifié ayant jusqu'à 6 atomes de carbone,
R⁵ a la définition indiquée ci-dessus pour R³ et est identique à R³ ou en est différent, ou représente
de l'hydrogène, un groupe cyano, carboxy ou un groupe alkoxycarbonyle linéaire ou ramifié ayant jusqu'à 6 atomes de carbone, ou bien
un groupe alkyle linéaire ou ramifié ayant jusqu'à 6 atomes de carbone, qui est substitué, le cas échéant par du fluor, du chlore, du brome, un radical hydroxy ou un radical alkoxy linéaire ou ramifié ayant jusqu'à 6 atomes de carbone, ou qui est substitué par un groupe de formule -O-(CH₂₎ₐ-R¹¹ ou -O-CO-R¹²,
dans laquelle
a est le nombre 0 ou 1,
et
R¹¹ est un groupe phényle ou un groupe benzyle, qui sont substitués, le cas échéant, dans la partie aromatique par du fluor, du chlore, du brome, un radical trifluorométhyle, cyano, nitro ou un radical alkyle linéaire ou ramifié ayant jusqu'à 4 atomes de carbone,
R¹² a la définition indiquée ci-dessus pour R¹¹ et est identique à R¹¹ ou en est différent, ou représente
un groupe alkyle linéaire ou ramifié ayant jusqu'à 6 atomes de carbone,
ou bien
R⁵ est un reste de formule -CH=N-O-R¹³ dans laquelle
R¹³ a la définition indiquée ci-dessus pour R¹² et est identique à R¹² ou en est différent.

3. 4-phényl-pyridones et 4-phényl-2-alkoxypyridines substituées suivant la revendication 1, dans lesquelles
R¹ est un groupe alkyle linéaire ou ramifié ayant jusqu'à 4 atomes de carbone,
R² est un groupe cyclopropyle ou un groupe alkyle linéaire ou ramifié ayant jusqu'à 4 atomes de carbone,
R³ représente un reste de formule 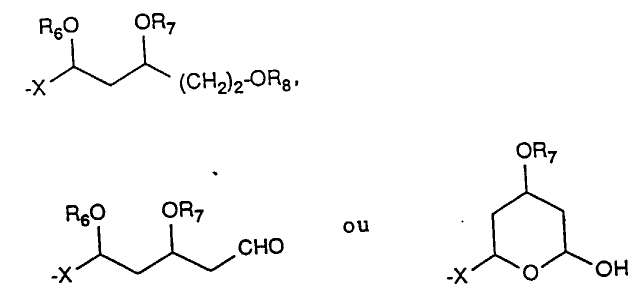
dans laquelle
X représente un groupe -CH₂-CH₂- ou -CH=CH-,
R⁶, R⁷ et R⁸ sont identiques ou différents et représentent de l'hydrogène ou un reste de formule -CO-R⁹ dans laquelle
R⁹ est un groupe alkyle linéaire ou ramifié ayant jusqu'à 4 atomes de carbone,
R⁴ est un groupe phényle qui est substitué, le cas échéant par du fluor, un radical trifluorométhyle ou un radical alkyle linéaire ou ramifié ayant jusqu'à 4 atomes de carbone,
R⁵ a la définition indiquée ci-dessus pour R³ et est identique à R³ ou en est différent, ou représente
de l'hydrogène ou
un groupe alkyle linéaire ou ramifié ayant jusqu'à 4 atomes de carbone, qui est substitué, le cas échéant par un radical hydroxy ou par un radical alkoxy linéaire ou ramifié ayant jusqu'à 4 atomes de carbone.

4. 4-phényl-pyridones et 4-phényl-2-alkoxypyridines substituées suivant la revendication 1, dans lesquelles
R¹ est un groupe alkyle linéaire ou ramifié ayant jusqu'à 4 atomes de carbone,
R² est un groupe cyclopropyle ou un groupe alkyle linéaire ou ramifié ayant jusqu'à 4 atomes de carbone,
R³ est un reste de formule 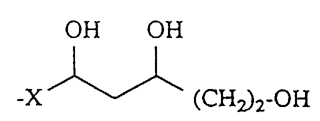
dans laquelle
X représente le groupe -CH₂-CH₂- ou -CH=CH-,
R⁴ est un groupe phényle qui est substitué, le cas échéant par du fluor, et
R⁵ a la définition indiquée ci-dessus pour R³ ou représente
un groupe alkyle linéaire ou ramifié ayant jusqu'à 4 atomes de carbone, qui est substitué, le cas échéant par un radical hydroxy ou alkoxy ayant jusqu'à 4 atomes de carbone.

5. 4-phényl-pyridones et 4-phényl-2-alkoxypyridines substituées suivant la revendication 1, destinées à une application thérapeutique.

6. Procédé de production de 4-phényl-pyridones et de 4-phényl-2-alkoxypyridines substituées suivant la revendication 1, caractérisé en ce qu'on réduit des pyridones de formule générale II 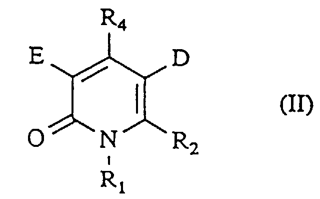 dans laquelle
D représente un reste de formule 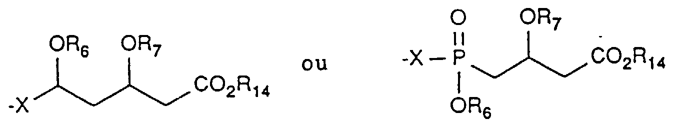
dans laquelle
R¹⁴ est un groupe alkyle en C₁ à C₆
dans des solvants inertes, sous une atmosphère de gaz protecteurs, le cas échéant en passant par le stade de l'aldéhyde, avec des agents réducteurs,
et au cas où -X- représente le groupe -CH₂-CH₂-, on hydrogène graduellement par des moyens classiques le groupe éthène (X = -CH=CH-) ou le groupe éthyne (X = -C≡C-) et on sépare éventuellement les isomères.

7. Médicament contenant au moins une 4-phénylpyridone ou une 4-phényl-2-alkoxypyridine substituée suivant la revendication 1.

8. Médicament suivant la revendication 1, destiné au traitement de l'hyperlipoprotéinémie.

9. Procédé de préparation d'un médicament suivant la revendication 7, caractérisé en ce que l'on fait prendre une forme administrable appropriée à des 4-phényl-pyridones ou des 4-phényl-2-alkoxypyridines substituées, le cas échéant à l'aide de substances auxiliaires et de supports classiques.

10. Utilisation de 4-phényl-pyridones et de 4-phényl-2-alkoxypyridines substituées pour la préparation de médicaments.
